# EUROPEAN PATENT APPLICATION

(11) **EP 4 663 143 A1**
(43) Date of publication of application: **17.12.2025**
(21) Application number: 24763444.7
(22) Date of filing: 26.01.2024
(51) Int. Cl.: A61B 18/14

(54) **MEDICAL DEVICE, MEDICAL DEVICE PACKAGE, METHOD FOR USING MEDICAL DEVICE, AND METHOD FOR PRODUCING MEDICAL DEVICE**

(30) Priority: 28.02.2023 JP 2023029328
(71) Applicant: Terumo Kabushiki Kaisha, Tokyo 151-0072 (JP)
(72) Inventor: TAKAHASHI, Yusuke, Ashigarakami-gun, Kanagawa 259-0151 (JP)
(74) Representative: Prüfer & Partner mbB Patentanwälte · Rechtsanwälte
(86) International application number: PCT/JP2024/002343
(87) International publication number: WO 2024/180959

(57) **Abstract**

A medical device and a method for using the medical device capable of reducing a possibility of breakage of an expansion body when the expansion body is housed in an inserter are provided. The medical device 10 includes an elongated shaft portion 20, a handheld operation portion 23 disposed at a proximal portion of the shaft portion 20, an expansion body 21 disposed at a distal portion of the shaft portion 20, and an inserter 25 including a lumen 65 through which the shaft portion 20 is inserted and which is capable of housing the expansion body 21, and the inserter 25 includes an inserter tube 60 that forms the lumen 65, a valve body 62 that abuts on an outer peripheral surface of the inserted shaft portion 20, and an injection port 63 that communicates with the lumen 65, and a length of the inserter tube 60 of the inserter 25 along an axial direction is equal to or greater than a length of the expansion body 21 along the axial direction and equal to or less than 150 mm, and, the inserter 25 is disposed in advance between the expansion body 21 and the handheld operation portion 23 in a state where the shaft portion 20 is inserted into the lumen 65 of the inserter 25.

## Description

### Technical Field

The present invention relates to a medical device including an expansion body at a distal portion of an elongated shaft portion, a medical device package, a method for using the medical device, and a method for manufacturing the medical device.

### Background Art

As a medical device, a medical device including an expansion body for performing treatment, or the like, at a distal portion of an elongated shaft portion is known. As a medical device including an expansion body, for example, a medical device is known that includes an electrode portion disposed in the expansion body that expands and contracts inside a living body, and performs treatment by ablation to cauterize a biological tissue by a high-frequency current from the electrode portion.

The shaft portion of the medical device is inserted into a delivery sheath inserted into the living body in advance. In this event, an inserter is used to insert the expansion body at the distal portion into the delivery sheath. The inserter includes an inserter shaft having an outer shape that is insertable into the delivery sheath, and the expansion body and the shaft portion are inserted into a lumen of the inserter shaft. As such a medical device, for example, there is a medical device described in Patent Literature 1.

### Citation List

### Patent Literature

Patent Literature 1: WO 2017-192477

### Summary of Invention

### Technical Problem

An expansion body is inserted into a proximal opening of an inserter from a distal portion in a reduced diameter state. Thus, an operator needs to insert the expansion body into the inserter while reducing the diameter with the fingers. When the diameter of the expansion body is reduced with the fingers, load is applied, which may lead to breakage.

The present invention has been made to solve the above-described problem, and an object of the present invention is to provide a medical device, a medical device package, a method for using the medical device, and a method for manufacturing the medical device capable of reducing a possibility of breakage of an expansion body when the expansion body is housed in an inserter.

### Solution to Problem

(1) A medical device according to the present invention that achieves the above object includes: an elongated shaft portion; a handheld operation portion disposed at a proximal portion of the shaft portion; an expansion body disposed at a distal portion of the shaft portion; and an inserter including a lumen through which the shaft portion is inserted and which is capable of housing the expansion body, in which the inserter includes: an inserter tube that forms the lumen; a valve body that abuts on an outer peripheral surface of the inserted shaft portion; and an injection port that communicates with the lumen, and a length of the inserter tube of the inserter along an axial direction is equal to or greater than a length of the expansion body along the axial direction and equal to or less than 150 mm, and the inserter is disposed in advance between the expansion body and the handheld operation portion in a state where the shaft portion is inserted into the lumen of the inserter.

(6) A medical device package according to the present invention that achieves the above object includes: the medical device; and a package that packages the medical device, in which the package is subjected to sterilization treatment in a state where the medical package in which the inserter is disposed in advance between the expansion body and the handheld operation portion is packaged by inserting the shaft portion into the lumen of the inserter.

(7) A method for using a medical device according to the present invention for achieving the above object is a method for using a medical device including an elongated shaft portion, a handheld operation portion disposed at a proximal portion of the shaft portion, an expansion body disposed at a distal portion of the shaft portion, and an inserter including a lumen through which the shaft portion is inserted and which is capable of housing the expansion body, in which the inserter is disposed in advance between the expansion body and the handheld operation portion in a state where the shaft portion is inserted, and the inserter is moved from a proximal side to a distal side to draw the expansion body in a distal opening of the inserter, and the expansion body is contracted and housed in the lumen of the inserter.

(8) A method for manufacturing a medical device according to the present invention to achieve the above object is a method for manufacturing a medical device including an elongated shaft portion, a handheld operation portion disposed at a proximal portion of the shaft portion, an expansion body disposed at a distal portion of the shaft portion, and an inserter including a lumen through which the shaft portion is inserted and which is capable of housing the expansion body, the method including: inserting the shaft portion to which the expansion body is attached at the distal portion into the lumen of the inserter from a distal side with respect to the inserter; and attaching a shaft connecting portion for connecting the shaft portion to the handheld operation portion to the proximal portion of the shaft portion.

(9) A method for manufacturing a medical device according to the present invention to achieve the above object is a method for manufacturing a medical device including an elongated shaft portion, a handheld operation portion disposed at a proximal portion of the shaft portion, an expansion body disposed at a distal portion of the shaft portion, and an inserter including a lumen through which the shaft portion is inserted and which is capable of housing the expansion body, the method including: inserting the shaft portion to which a shaft connecting portion for connecting the shaft portion to the handheld operation portion is attached at the proximal portion into the lumen of the inserter from a proximal side of the inserter; and attaching the expansion body to the distal portion of the shaft portion.

(10) A method for manufacturing a medical device according to the present invention to achieve the above object is a method for manufacturing a medical device including an elongated shaft portion, a handheld operation portion disposed at a proximal portion of the shaft portion, an expansion body disposed at a distal portion of the shaft portion, and an inserter including a lumen through which the shaft portion is inserted and which is capable of housing the expansion body, the method including: inserting the shaft portion into the lumen of the inserter; and attaching a shaft connecting portion for connecting the shaft portion to the handheld operation portion to the proximal portion of the shaft portion inserted into the lumen of the inserter, and attaching the expansion body to the distal portion of the shaft portion inserted into the lumen of the inserter.

### Advantageous Effects of Invention

In the medical device configured as described above, the inserter is disposed in advance between the expansion body and the handheld operation portion, and thus, the expansion body can be drawn from the distal opening of the inserter while being contracted in the radial direction. Thus, in the medical device, when the expansion body is housed in the inserter, it is not necessary for an operator to manually contract the expansion body, and it is possible to reduce a risk of breakage of the expansion body by load.

(2) In the medical device of (1), the lumen of the inserter may have a small diameter portion protruding from an inner wall to a center side on a distal side of the valve body, and the small diameter portion may have an inner diameter smaller than an outer diameter of the proximal portion of the expansion body. This makes it possible to prevent the expansion body from falling off from the inserter in the medical device.

(3) In the medical device of (1) or (2), at least part of the inserter tube may have flexibility. As a result, when the expansion body is housed in the lumen, the medical device can be curved following the shaft portion curved in the vicinity of the expansion body, so that it is possible to reduce the load applied to the shaft portion and the expansion body and reduce a risk of breakage.

(4) In the medical device according to any one of (1) to (3), the inserter may include an inserter hub on a proximal side of the inserter tube, and the valve body may be housed inside the inserter hub. By this means, the inserter can reliably hold the valve body.

(5) In the medical device according to any one of (1) to (4), the inserter tube may be formed with a transparent or translucent resin. This makes it possible to visually confirm air remaining in the lumen at the time of injecting liquid into the inserter in the medical device.

In the medical device package configured as described above, the inserter is disposed in advance between the expansion body and the handheld operation portion in a state where the operator takes out the medical device from the package, and the operator can draw the expansion body while contracting the expansion body in the radial direction from the distal opening of the inserter.

In the method for using the medical device configured as described above, the expansion body is drawn from the distal opening of the inserter while being contracted in the radial direction, and thus, when the expansion body is housed in the inserter, it is not necessary for the operator to contract the expansion body by hand, and it is possible to reduce a risk of breakage of the expansion body by load.

In the method for manufacturing the medical device configured as described above, it is possible to manufacture the medical device in which the inserter is disposed in advance between the expansion body and the handheld operation portion, so that the operator can draw the expansion body while contracting the expansion body in the radial direction from the distal opening of the inserter.

### Brief Description of Drawings

Fig. 1 is a front view illustrating an overall configuration of a medical device according to an embodiment.
Fig. 2 is an enlarged perspective view illustrating the vicinity of an expansion body.
Fig. 3 is an enlarged front view of the vicinity of the expansion body.
Fig. 4 is an enlarged cross-sectional view of the vicinity of an inserter in the medical device.
Fig. 5 is a front view illustrating a process of drawing the expansion body into a lumen of the inserter.
Fig. 6 is an explanatory diagram schematically illustrating a state where the expansion body is disposed in an atrial septum, including a front view of the medical device and a cross-sectional view of a biological tissue.
Fig. 7 is an enlarged cross-sectional view of the vicinity of a connecting portion between an inserter and a delivery sheath according to a modification, (a) is a view illustrating a state before connection and (b) is a view illustrating a state after connection.
Fig. 8 is a front view of a medical device package.
Fig. 9 is a view illustrating a manufacturing process of the medical device, (a) is a view illustrating a state before a shaft portion to which the expansion body is attached is inserted into the inserter, (b) is a view illustrating a state before a shaft connecting portion is attached to the shaft portion inserted into the inserter, and (c) is a view illustrating a state in which a traction shaft is inserted into the shaft portion.
Fig. 10 is a view illustrating a manufacturing process of the medical device, (a) is a view illustrating a state before the shaft portion to which the shaft connecting portion is attached is inserted into the inserter, (b) is a view illustrating a state before the expansion body is attached to the shaft portion inserted into the inserter, and (c) is a view illustrating a state in which the traction shaft is inserted into the shaft portion.
Fig. 11 is a view illustrating a manufacturing process of the medical device, (a) is a view illustrating a state before the shaft portion is inserted into the inserter, (b) is a view illustrating a state before the shaft connecting portion and the expansion body are attached to the shaft portion inserted into the inserter, and (c) is a view illustrating a state in which the traction shaft is inserted into the shaft portion.

### Description of Embodiments

Hereinafter, an embodiment of the present invention will be described with reference to the drawings. Note that dimensional ratios in the drawings may be exaggerated and different from actual ratios for convenience of description. Furthermore, in the present specification, a side of a medical device 10 to be inserted into a living body will be referred to as a "distal end" or a "distal side", and a hand side to be operated will be referred to as a "proximal end" or a "proximal side".

The medical device according to the embodiment described below is configured to expand a puncture hole Hh formed in an atrial septum HA of a heart H of a patient, and to further perform maintenance treatment to maintain the expanded puncture hole Hh at the increased size.

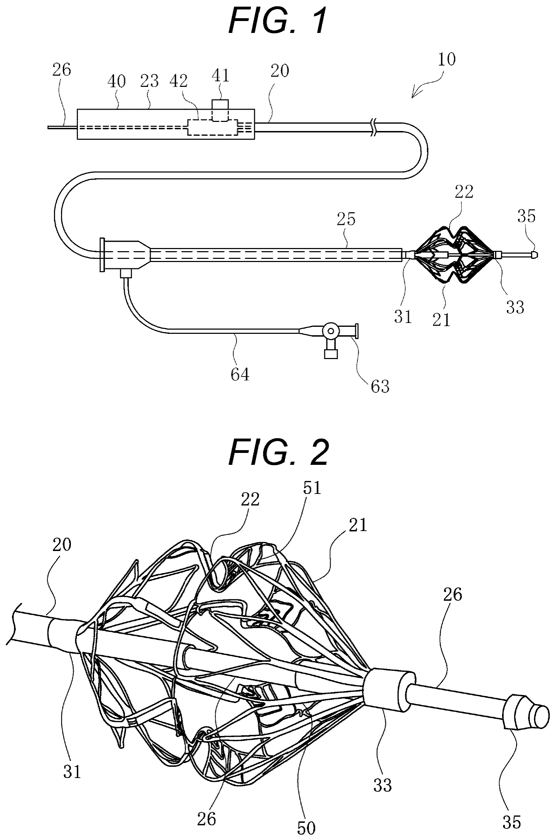

As illustrated in Fig. 1, the medical device 10 according to the present embodiment includes an elongated shaft portion 20, an expansion body 21 disposed at a distal portion of the shaft portion 20, and a handheld operation portion 23 disposed at a proximal portion of the shaft portion 20. The expansion body 21 has an electrode portion 22, which is an energy transfer element for performing the above-described maintenance treatment. The expansion body 21 is connected to the shaft portion 20 at a connecting portion 31 at the proximal end. A length of the shaft portion 20 along the axial direction is a range of equal to or greater than 850 mm and equal to or less than 1500 mm. A length of the expansion body 21 along the axial direction is a range of equal to or greater than 15 mm and equal to or less than 150 mm. Note that the lengths of the shaft portion 20 and the expansion body 21 along the axial direction may be out of this range.

The medical device 10 includes an inserter 25 through which the shaft portion 20 is inserted. The inserter 25 is disposed in advance between the expansion body 21 and the handheld operation portion 23 in a state where the shaft portion 20 is inserted. The shaft portion 20 can move along the axial direction with respect to the inserter 25.

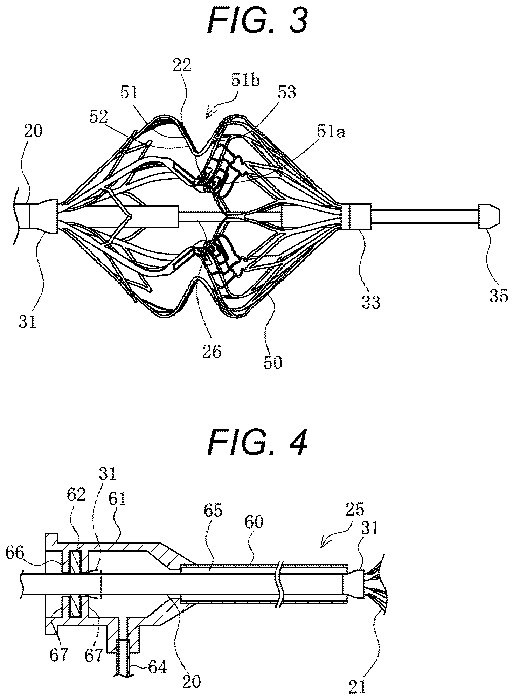

As illustrated in Figs. 2 and 3, a traction shaft 26 is disposed in the shaft portion 20 so as to be slidable with respect to the shaft portion 20. The traction shaft 26 is disposed from a position proximal of the handheld operation portion 23 to a position distal of the expansion body 21. The traction shaft 26 passes through an inside of the expansion body 21, further passes through a distal fixing portion 33 to which a distal end of the expansion body 21 is fixed, and protrudes from the distal end of the expansion body 21. A distal portion of the traction shaft 26 is fixed to a distal member 35.

The distal member 35 to which the distal portion of the traction shaft 26 is fixed needs not be fixed to the expansion body 21. As a result, when the traction shaft 26 slides in a proximal direction with respect to the shaft portion 20, the distal member 35 can apply compressing force to the expansion body 21 along an axial center of the shaft portion 20. In addition, when the expansion body 21 is housed in the inserter 25, the distal member 35 is separated to the distal side from the expansion body 21. Accordingly, the expansion body 21 can be rather easily moved in the axial direction, which can improve housing capability.

As illustrated in Fig. 1, the handheld operation portion 23 includes a housing 40 to be held by an operator, an operation dial 41 that can be rotationally operated by the operator, and a conversion mechanism 42 that operates in conjunction with the rotation of the operation dial 41. The traction shaft 26 is held by the conversion mechanism 42 inside the handheld operation portion 23. The conversion mechanism 42 can move the held traction shaft 26 forward and rearward in the axial direction in conjunction with the rotation of the operation dial 41. For example, a rack and pinion mechanism can be used as the conversion mechanism 42. In addition, the handheld operation portion 23 may have a structure in which a guide rail is provided in the housing 40 and an operation portion with which the traction shaft 26 can be operated in the axial direction along the guide rail is provided.

It is preferable that the shaft portion 20 is formed with a material having a certain degree of flexibility. Examples of such a material include polyolefin such as polyethylene, polypropylene, polybutene, ethylene-propylene copolymer, ethylene-vinyl acetate copolymer, ionomer, or a mixture of two or more of them, soft polyvinyl chloride resin, polyamide, polyamide elastomer, polyester, polyester elastomer, polyurethane, fluorine resin such as polytetrafluoroethylene, polyimide, PEEK, silicone rubber, and latex rubber.

The traction shaft 26 can be formed with, for example, an elongated tubular material including a super elasticity alloy such as a nickel-titanium alloy and a copper-zinc alloy, a metal material such as stainless steel, a resin material having comparatively high rigidity, or the like. A lumen for inserting a guide wire is formed inside the traction shaft 26 formed with an elongated tubular material. The traction shaft 26 may be formed with a braided reinforced tube in which a metal wire reinforcing material is embedded in a resin tube.

The distal member 35 can be formed with, for example, a super elasticity alloy such as a nickel-titanium alloy or a copper-zinc alloy, a metal material such as stainless steel, a polymer material such as polyolefin, polyvinyl chloride, polyamide, polyamide elastomer, polyurethane, polyurethane elastomer, polyimide, and fluororesin or a mixture of the above polymer materials. Alternatively, the distal member 35 can be formed with a multilayer tube containing two or more kinds of polymer materials.

The expansion body 21 includes a plurality of wire portions 50 in a circumferential direction. The wire portions 50 form a mesh-shaped structure by branching and joining along a length direction. As a result, the expansion body 21 can expand and contract in a radial direction. Proximal portions of the wire portions 50 extend from the connecting portion 31 to the distal side. Distal portions of the wire portion 50 extend from the proximal portion of the distal fixing portion 33 to the proximal side. The wire portions 50 are inclined to increase in the radial direction from both end portions toward central portions in the axial direction. Further, the wire portion 50 includes a recess 51 in the central portion in the axial direction, the recess 51 being recessed radially inward of the expansion body 21. An innermost portion of the recess 51 in the radial direction is a bottom portion 51a. The recess 51 defines a reception space 51b that can receive a biological tissue when the expansion body 21 expands.

The recess 51 includes a proximal side upright portion 52 extending radially outward from the proximal end of the bottom portion 51a and a distal side upright portion 53 extending radially outward from the distal end of the bottom portion 51a. When the traction shaft 26 slides in a proximal direction with respect to the shaft portion 20 and compressing force is applied to the expansion body 21, the distal side upright portion 53 and the proximal side upright portion 52 approach each other, and both portions come in close contact with the biological tissue received in the reception space 51b. An electrode portion 22 is disposed along the recess 51 to face the reception space 51b, in the proximal side upright portion 52. In other words, the electrode portion 22 is disposed along the expansion body 21 in an intermediate portion in a central axis direction of the expansion body 21. In the present embodiment, six electrode portions 22 are disposed in the circumferential direction at substantially equal intervals. Note that the electrode portion 22 may be disposed in the distal side upright portion 53. Note that the number of the electrode portions 22 may be less than six or more than six (for example, ten) at substantially equal intervals along the circumferential direction by changing the shape of the wire portion 50.

The wire portions 50 forming the expansion body 21 can be formed by cutting a single metal cylindrical member with laser, or the like. The wire portion 50 can be formed with a metal material. Examples of the metal material that may be used include a titanium-based (Ti-Ni, Ti-Pd, Ti-Nb-Sn, etc.) alloy, a copper-based alloy, stainless steel, β-titanium steel, and a Co-Cr alloy. Note that an alloy having spring property such as a nickel titanium alloy may also be used as the material. However, a material of the wire portion 50 is not limited to the above materials, and the wire portion 50 may be formed with other materials.

The electrode portion 22 is connected to an energy supply device (not illustrated), which is an external device. A high-frequency voltage is applied from the energy supply device to an electrode pair including two electrode portions 22, and energy is applied between them. In other words, the electrode portion 22 is configured as a bipolar electrode.

As illustrated in Fig. 4, the inserter 25 includes an inserter tube 60 forming a lumen 65 through which the shaft portion 20 is inserted and which is capable of housing the expansion body 21, and an inserter hub 61 provided on the proximal side of the inserter tube 60. A length of the inserter tube 60 along the axial direction is equal to or greater than a length of the expansion body 21 along the axial direction and is equal to or less than 150 mm. In other words, the length of the inserter tube 60 along the axial direction is shorter than a length of the shaft portion 20 along the axial direction. Thus, the inserter 25 houses the distal portion of the shaft portion 20 including the expansion body 21 and is used to insert the expansion body 21 into the delivery sheath 70. The inserter tube 60 can be formed with a material similar to that of the shaft portion 20. The inserter tube 60 has flexibility and can be curved by receiving force. Note that part of the inserter tube 60 on the distal side along the axial direction may have flexibility.

The inserter hub 61 includes a valve body 62 therein. The valve body 62 abuts on an outer peripheral surface of the shaft portion 20 inserted into the inserter 25. As the valve body 62, for example, a substantially elliptical or substantially circular membrane body formed with silicone rubber, latex rubber, butyl rubber, isoprene rubber, or the like, which is an elastic member, can be used.

The inserter hub 61 includes a holding portion 66 for holding the valve body 62. The holding portion 66 is formed with two small diameter portions 67 protruding from an inner wall of the inserter hub 61 toward the center side. One of the small diameter portions 67 is disposed on the distal side of the valve body 62 in the lumen 65 and has an inner diameter smaller than an outer shape of the connecting portion 31 disposed at the proximal portion of the expansion body 21. Thus, in a case where the shaft portion 20 moves to the proximal side with respect to the inserter 25, the connecting portion 31 cannot pass through the small diameter portion 67 as indicated by a one-dot chain line in Fig. 4. Accordingly, the inserter 25 can be prevented from falling off from the shaft portion 20.

The inserter 25 includes an injection port 63 that communicates with the lumen 65 (see Fig. 1). The injection port 63 and the lumen 65 communicate with each other by a communication tube 64. A liquid injection device such as a syringe can be connected to the injection port 63, so that liquid can be injected into the lumen 65.

A method for using the medical device 10 will be described. The medical device 10 is used in treatment to be performed on a patient suffering from chronic heart failure in which myocardial hypertrophy appears in a left ventricle of the heart H and stiffness (hardness) increases so that a blood pressure increases in a left atrium HLa.

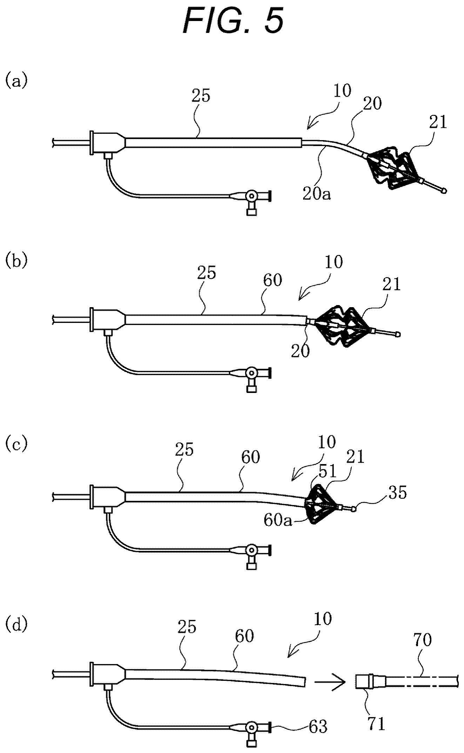

As illustrated in Fig. 5(a), in the medical device 10 before use, the inserter 25 is assembled in advance to the shaft portion 20, and the expansion body 21 is exposed to the outside of the inserter 25. The operator houses the expansion body 21 in the inserter 25 before inserting the expansion body 21 and the shaft portion 20 into the delivery sheath 70.

The shaft portion 20 exposed to the distal side of the inserter 25 has a curved portion 20a curved in one direction in the vicinity of the expansion body 21. The shaft portion 20 has the curved portion 20a, and thus, when the expansion body 21 is disposed in the atrial septum HA in the heart H, a direction of the expansion body 21 can be easily adjusted to the atrial septum HA.

As illustrated in Fig. 5(b), the operator moves the inserter 25 from the proximal side to the distal side with respect to the shaft portion 20. As a result, the distal portion of the shaft portion 20 is housed in the lumen 65 of the inserter tube 60. The shaft portion 20 has the curved portion 20a, and thus, the inserter tube 60 having flexibility is gradually curved following the curved portion 20a.

When the operator moves the inserter 25 further to the proximal side, as illustrated in Fig. 5(c), the expansion body 21 is drawn into the distal opening 60a of the inserter tube 60 while contracting in the radial direction. In Fig. 5(c), the expansion body 21 is in a state where a portion on the proximal side of the recess 51 is drawn into the inserter tube 60. In this event, the expansion body 21 expands along the axial direction and the distal end of the expansion body 21 approaches the distal member 35. Further, the inserter tube 60 is further curved following the curved portion 20a of the shaft portion 20.

If the operator moves the inserter 25 further to the proximal side, as illustrated in Fig. 5(d), the expansion body 21 is housed in the inserter tube 60. The inserter tube 60 housing the expansion body 21 is curved by the curved portion 20a of the shaft portion 20. The inserter tube 60 has flexibility and can be curved following the curved portion 20a of the shaft portion 20, and thus, the shaft portion 20 is not excessively linearly deformed, so that it is possible to reduce a risk of breakage of the shaft portion 20. In addition, the shaft portion 20 is not excessively deformed, so that it is possible to prevent the expansion body 21 from being drawn into the inserter 25 in a state where load is applied to one side of the expansion body 21, and thus, it is possible to reduce a risk of breakage of the expansion body 21.

The inserter 25 is disposed in advance between the expansion body 21 and the handheld operation portion 23, and thus, the expansion body 21 can be drawn from the distal opening 60a of the inserter 25 while being contracted in the radial direction. Thus, in the medical device 10 of the present embodiment, when the expansion body 21 is housed in the inserter 25, it is not necessary for the operator to contract the expansion body 21 by hand, and it is possible to reduce a risk of breakage of the expansion body 21 by load.

After the expansion body 21 is housed in the lumen 65 of the inserter tube 60, the operator injects liquid from the injection port 63 into the lumen 65. The liquid to be injected can be saline. The liquid to be injected may be other than saline. Accordingly, air is removed from the inside of the inserter 25. The length of the inserter tube 60 along the axial direction is equal to or greater than the length of the expansion body 21 along the axial direction and is equal to or less than 150 mm, which is short, so that it is possible to reduce an amount of the liquid to be injected and shorten a period required for injection. Note that the inserter tube 60 is preferably formed with a transparent or translucent resin. As a result, air remaining in the lumen 65 can be visually confirmed at the time of injecting the liquid into the inserter 60. It is therefore possible to reduce a risk that air remaining in the inserter 60 is injected into the living body to cause peripheral emboli.

After air is removed from the inside of the inserter 25, the operator inserts the inserter 25 into a hub 71 of the delivery sheath 70 that inserted in advance into the living body. Once the inserter 25 is inserted into the delivery sheath 70, the operator moves the shaft portion 20 to the distal side and pushes the expansion body 21 into the delivery sheath 70.

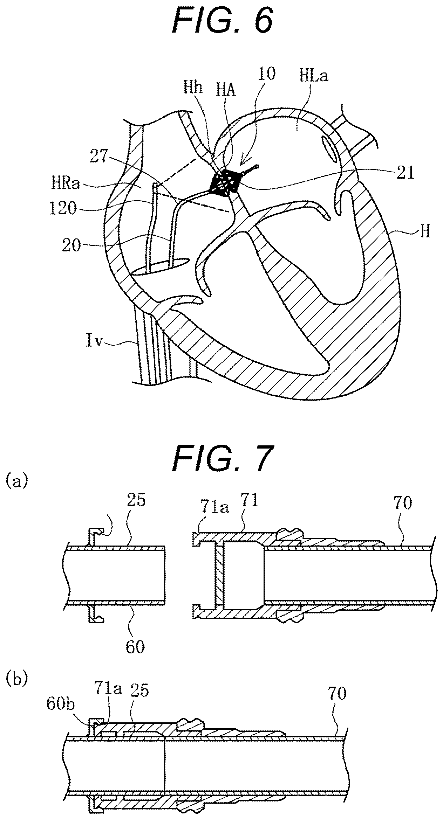

As illustrated in Fig. 6, the expansion body 21 is delivered from an inferior vena cava Iv to the vicinity of the atrial septum HA via a right atrium HRa, and is disposed at a position of the puncture hole Hh which is formed in advance. The operator inserts the delivery sheath 70 until the distal portion of the delivery sheath 70 penetrates the atrial septum HA and reaches a left atrium HLa. Thereafter, the operator moves the delivery sheath 70 to the proximal side to expose the expansion body 21. In this manner, a diameter of the expansion body 21 increases, and the recess 51 is disposed in the puncture hole Hh of the atrial septum HA and receives the biological tissue surrounding the puncture hole Hh in the reception space 51b. The shaft portion 20 includes the curved portion 27 as described above, and the curved portion 27 can be exposed from the delivery sheath 70, thereby can be curved in one direction. This allows the expansion body 21 to be disposed in such a manner that the axial direction thereof is close to perpendicular to a plane of the atrial septum HA.

By disposing the expansion body 21 at the atrial septum HA and applying high-frequency energy to an edge of the puncture hole Hh via the electrode portion 22 in a state where the electrode portion 22 is pressed against the biological tissue, the edge of the puncture hole Hh can be cauterized (heated and cauterized) with the high-frequency energy. The high-frequency energy is applied by applying a voltage between the pair of electrode portions 22 adjacent to each other in the circumferential direction. This results in making it possible to inhibit blockage due to natural healing of the puncture hole Hh and maintain a size thereof.

When the medical device 10 is used, hemodynamics is checked by a hemodynamics checking device 120 delivered to the right atrium HRa via the inferior vena cava Iv. As the hemodynamics checking device 120, a known echo catheter can be used, for example. The operator can display an echo image acquired by the hemodynamics checking device 120 on a display device such as a display and can check an amount of blood passing through the puncture hole Hh based on the displayed result.

A modification of the inserter 25 will be described. As illustrated in Fig. 7(a), the inserter tube 60 of the inserter 25 may have an engagement claw portion 60b functioning as a first engagement portion at the distal portion. In this case, the hub 71 of the delivery sheath 70 has an engagement recess portion 71a that can function as a second engagement portion at the proximal portion. As illustrated in Fig. 7(b), by engaging the engagement claw portion 60b which is the first engagement portion of the inserter 25 with the engagement recess portion 71a which is the second engagement portion of the delivery sheath 70, the inserter 25 can be fixed to the delivery sheath 70 in a state where the inserter tube 60 is inserted into the delivery sheath 70. This makes it easier to insert the expansion body 21 into the delivery sheath 70. Note that one of the first engagement portion and the second engagement portion may be a male screw portion, and the other may be a female screw portion.

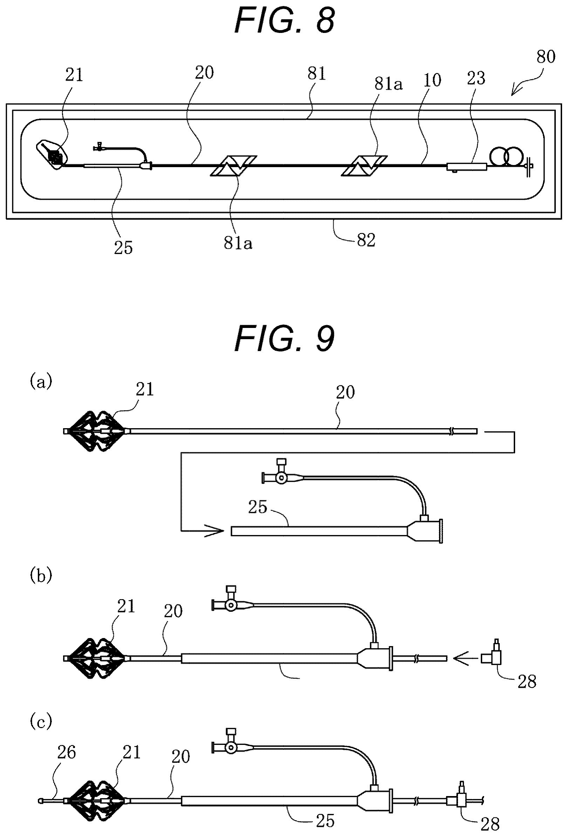

The medical device package 80 that packages the medical device 10 will be described. As illustrated in Fig. 8, the medical device package 80 is formed by packaging a mount 81 holding the medical device 10 with a package 82. The package 82 is subjected to sterilization treatment in a state where the medical device 10 in which the inserter 25 is disposed in advance between the expansion body 21 and the handheld operation portion 23 is packaged by inserting the shaft portion 20 into the lumen 65 of the inserter 25. The mount 81 includes a fixing portion 81a that holds the shaft portion 20 of the medical device 10. The package 82 is formed with a plastic sheet on one surface and a gas-permeable nonwoven fabric on the other surface so as to enable gas sterilization such as ethylene oxide gas sterilization.

A method for manufacturing the medical device 10 will be described next. As illustrated in Fig. 9(a), the expansion body 21 is attached to the distal portion of the shaft portion 20 in advance, and the shaft portion 20 is inserted into the lumen 65 of the inserter 25 from the distal side with respect to the inserter 25. After the shaft portion 20 is inserted until the distal end of the inserter 25 is positioned in the vicinity of the expansion body 21, the shaft connecting portion 28 is attached to the proximal portion of the shaft portion 20 as illustrated in Fig. 9(b). The shaft connecting portion 28 is a component for connecting the shaft portion 20 to the handheld operation portion 23. Next, as illustrated in Fig. 9(c), the traction shaft 26 is inserted from the distal side of the expansion body 21 and inserted into the shaft portion 20. Thereafter, the handheld operation portion 23 is attached to the position of the shaft connecting portion 28. Note that the traction shaft 26 may be inserted into the shaft portion 20 from the distal side of the expansion body 21 before the inserter 25 is inserted into the shaft portion 20.

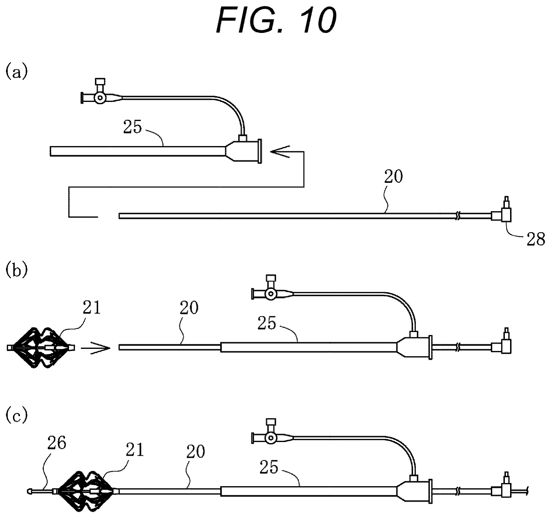

Another method for manufacturing the medical device 10 will be described. As illustrated in Fig. 10(a), the shaft connecting portion 28 is attached to the proximal portion of the shaft portion 20 in advance, and the shaft portion 20 is inserted into the lumen 65 of the inserter 25 from the proximal side with respect to the inserter 25. After the inserter 25 is inserted to a predetermined position, the expansion body 21 is attached to the distal portion of the shaft portion 20 as illustrated in Fig. 10(b). Next, as illustrated in Fig. 10(c), the traction shaft 26 is inserted from the distal side of the expansion body 21 and inserted into the shaft portion 20. Note that the traction shaft 26 may be inserted into the expansion body 21 in advance before the expansion body 21 is attached to the distal portion of the shaft portion 20, and may be inserted into the shaft portion 20 from the distal side when the expansion body 21 is attached to the distal portion of the shaft portion 20. Thereafter, the handheld operation portion 23 is attached to the position of the shaft connecting portion 28.

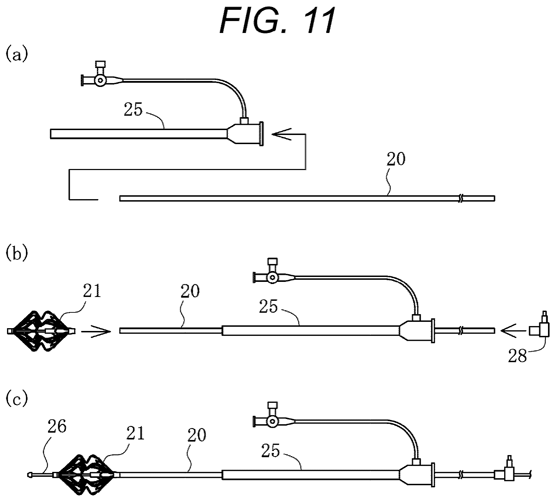

A still another method for manufacturing the medical device 10 will be described. As illustrated in Fig. 11(a), the shaft portion 20 is inserted into the lumen 65 of the inserter 25. The shaft portion 20 can be inserted from either the proximal portion or the distal portion with respect to the inserter 25. When the shaft portion 20 is inserted into the inserter 25, as illustrated in Fig. 11(b), the shaft connecting portion 28 is attached to the proximal portion of the shaft portion 20, and the expansion body 21 is attached to the distal portion of the shaft portion 20. Next, as illustrated in Fig. 11(c), the traction shaft 26 is inserted from the distal side of the expansion body 21 and inserted into the shaft portion 20. Note that the traction shaft 26 may be inserted into the expansion body 21 in advance before the expansion body 21 is attached to the distal portion of the shaft portion 20, and may be inserted into the shaft portion 20 from the distal side when the expansion body 21 is attached to the distal portion of the shaft portion 20. Thereafter, the handheld operation portion 23 is attached to the position of the shaft connecting portion 28.

As described above, (1) the medical device 10 according to the present embodiment includes the elongated shaft portion 20, the handheld operation portion 23 disposed at the proximal portion of the shaft portion 20, the expansion body 21 disposed at the distal portion of the shaft portion 20, and the inserter 25 including the lumen 65 through which the shaft portion 20 is inserted and which is capable of housing the expansion body 21, the inserter 25 includes the inserter tube 60 that forms the lumen 65, the valve body 62 that abuts on the outer peripheral surface of the inserted shaft portion 20, and the injection port 63 that communicates with the lumen 65, and a length of the inserter tube 60 of the inserter 25 along the axial direction is equal to or greater than a length of the expansion body 21 along the axial direction and equal to or less than 150 mm, and the inserter 25 is disposed in advance between the expansion body 21 and the handheld operation portion 23 in a state where the shaft portion 20 is inserted into the lumen 65 of the inserter 25. In the medical device 10 configured in this manner, the inserter 25 is disposed in advance between the expansion body 21 and the handheld operation portion 23, and thus, the expansion body 21 can be drawn while being contracted in the radial direction from the distal opening of the inserter 25. Thus, in the medical device 10, when the expansion body 21 is housed in the inserter 25, it is not necessary for the operator to manually contract the expansion body 21, and it is possible to reduce a risk of breakage of the expansion body 21 by load.

(2) In the medical device 10 of (1), the lumen 65 of the inserter 25 may have the small diameter portion 67 protruding from the inner wall to the center side on the distal side of the valve body 62, and the small diameter portion 67 may have an inner diameter smaller than an outer diameter at the proximal portion of the expansion body 21. This makes it possible to prevent the expansion body 21 from falling off from the inserter 25 in the medical device 10.

(3) In the medical device 10 of (1) or (2), at least part of the inserter tube 60 may be flexible. As a result, when the expansion body 21 is housed in the lumen 65, the medical device 10 can be curved following the shaft portion 20 curved in the vicinity of the expansion body 21, so that it is possible to reduce load applied to the shaft portion 20 and the expansion body 21 and reduce a risk of breakage.

(4) In the medical device 10 of any one of (1) to (3), the inserter 25 may include the inserter hub 61 on the proximal side of the inserter tube 60, and the valve body 62 may be housed inside the inserter hub 61. Accordingly, the inserter 25 can reliably hold the valve body 62.

(5) In the medical device 10 according to any one of (1) to (4), the inserter tube 60 may be formed with a transparent or translucent resin. As a result, the medical device 10 can visually confirm air remaining in the lumen 65 when liquid is injected into the inserter 60.

(6) The medical device package 80 according to the present embodiment includes the medical device 10 and the package 82 for packaging the medical device 80, and the package 82 is subjected to sterilization treatment in a state where the medical device 10 in which the inserter 25 is disposed in advance between the expansion body 21 and the handheld operation portion 23 is packaged by inserting the shaft portion 20 into the lumen 65 of the inserter 25. In the medical device package 80 configured in this manner, the inserter 25 is disposed in advance between the expansion body 21 and the handheld operation portion 23 in a state where the operator takes out the medical device 10 from the package 82, and the operator can draw the expansion body 21 while contracting the expansion body 21 in the radial direction from the distal opening of the inserter 25.

(7) A method for using the medical device 10 according to the present embodiment is a method for using the medical device 10 including the elongated shaft portion 20, the handheld operation portion 23 disposed at the proximal portion of the shaft portion 20, the expansion body 21 disposed at the distal portion of the shaft portion 20, and the inserter 25 including the lumen 65 through which the shaft portion 20 is inserted and which is capable of housing the expansion body 21, in which the inserter 25 is disposed in advance between the expansion body 21 and the handheld operation portion 23 in a state where the shaft portion 20 is inserted, and the inserter 25 is moved from the proximal side to the distal side to draw the expansion body 21 into the distal opening of the inserter 25, and the expansion body 21 is contracted and housed in the lumen 65 of the inserter 25. In the method for using the medical device 10 configured in this manner, the expansion body 21 is drawn while being contracted in the radial direction from the distal opening of the inserter 25, when the expansion body 21 is housed in the inserter 25, it is not necessary for the operator to contract the expansion body 21 by hand, and it is possible to reduce a risk of breakage of the expansion body 21 by load.

(8) A method for manufacturing the medical device 10 according to the present embodiment is a method for manufacturing the medical device 10 including the elongated shaft portion 20, the handheld operation portion 23 disposed at the proximal portion of the shaft portion 20, the expansion body 21 disposed at the distal portion of the shaft portion 20, and the inserter 25 including the lumen 65 through which the shaft portion 20 is inserted and which is capable of housing the expansion body 21, the method including inserting the shaft portion 20 to which the expansion body 21 is attached at the distal portion into the lumen 65 of the inserter 25 from the distal side with respect to the inserter 25; and attaching the shaft connecting portion 28 for connecting the shaft portion 20 to the handheld operation portion 23 to the proximal portion of the shaft portion 20. In the method for manufacturing the medical device 10 configured in this manner, it is possible to manufacture the medical device 10 in which the inserter 25 is disposed in advance between the expansion body 21 and the handheld operation portion 23, so that the operator can draw the expansion body 21 while contracting the expansion body 21 in the radial direction from the distal opening of the inserter 25.

(9) A method for manufacturing the medical device 10 according to the present embodiment is a method for manufacturing the medical device 10 including the elongated shaft portion 20, the handheld operation portion 23 disposed at the proximal portion of the shaft portion 20, the expansion body 21 disposed at the distal portion of the shaft portion 20, and the inserter 25 including the lumen 65 through which the shaft portion 20 is inserted and which is capable of housing the expansion body 21, the method including: inserting the shaft portion 20 to which a shaft connecting portion 28 for connecting the shaft portion 20 to the handheld operation portion 23 is attached at the proximal portion into the lumen 65 of the inserter 25 from the proximal side of the inserter 25; and attaching the expansion body 21 to the distal portion of the shaft portion 20. In the method for manufacturing the medical device 10 configured in this manner, it is possible to manufacture the medical device 10 in which the inserter 25 is disposed in advance between the expansion body 21 and the handheld operation portion 23, so that the operator can draw the expansion body 21 while contracting the expansion body 21 in the radial direction from the distal opening of the inserter 25.

(1) A method for manufacturing the medical device 10 according to the present embodiment is a method for manufacturing the medical device 10 including the elongated shaft portion 20, the handheld operation portion 23 disposed at the proximal portion of the shaft portion 20, the expansion body 21 disposed at the distal portion of the shaft portion 20, and the inserter 25 including the lumen 65 through which the shaft portion 20 is inserted and which is capable of housing the expansion body 21, the method including: inserting the shaft portion 20 into the lumen 65 of the inserter 25; and attaching the shaft connecting portion 28 for connecting the shaft portion 20 to the handheld operation portion 23 to the proximal portion of the shaft portion 20 inserted into the lumen 65 of the inserter 25, and attaching the expansion body 21 to the distal portion of the shaft portion 20 inserted into the lumen 65 of the inserter 25. In the method for manufacturing the medical device 10 configured in this manner, it is possible to manufacture the medical device 10 in which the inserter 25 is disposed in advance between the expansion body 21 and the handheld operation portion 23, so that the operator can draw the expansion body 21 while contracting the expansion body 21 in the radial direction from the distal opening of the inserter 25.

Note that the present invention is not limited to the embodiment described above, and those skilled in the art can make various modifications within the technical idea of the present invention.

Note that the present application is based on Japanese Patent Application No. 2023-29328 filed on February 28, 2023, the entire disclosed content of which is incorporated herein by reference.

### Reference Signs List

- 10: Medical device
- 11: Guide wire
- 20: Shaft portion
- 21: Expansion body
- 22: Electrode portion (energy transfer portion)
- 23: Handheld operation portion
- 25: Inserter
- 26: Traction shaft
- 27: Curved portion
- 31: Connecting portion
- 33: Distal fixing portion
- 35: Distal member
- 40: Housing
- 41: Operation dial
- 42: Conversion mechanism
- 50: Wire portion
- 51: Recess
- 60: Inserter tube
- 60a: Distal opening
- 60b: Engagement claw portion
- 61: Inserter hub
- 62: Valve body
- 63: Injection port
- 64: Communication tube
- 65: Lumen
- 66: Holding portion
- 67: Small diameter portion
- 70: Delivery sheath
- 71: Hub
- 71a: Engagement recess

## Claims

1. A medical device comprising:
an elongated shaft portion;
a handheld operation portion disposed at a proximal portion of the shaft portion;
an expansion body disposed at a distal portion of the shaft portion; and
an inserter including a lumen through which the shaft portion is inserted and which is capable of housing the expansion body, wherein
the inserter includes an inserter tube that forms the lumen, a valve body that abuts on an outer peripheral surface of the inserted shaft portion, and an injection port that communicates with the lumen, and
a length of the inserter tube of the inserter along an axial direction is equal to or greater than a length of the expansion body along the axial direction and equal to or less than 150 mm, and the inserter is disposed in advance between the expansion body and the handheld operation portion in a state where the shaft portion is inserted into the lumen of the inserter.

2. The medical device according to claim 1, wherein
the lumen of the inserter has a small diameter portion protruding from an inner wall to a center side on a distal side of the valve body, and
the small diameter portion has an inner diameter smaller than an outer diameter of the proximal portion of the expansion body.

3. The medical device according to claim 1 or 2, wherein at least part of the inserter tube has flexibility.

4. The medical device according to claim 1 or 2, wherein
the inserter includes an inserter hub on a proximal side of the inserter tube, and
the valve body is housed inside the inserter hub.

5. The medical device according to claim 1 or 2, wherein the inserter tube is formed with a transparent or translucent resin.

6. A medical device package comprising:
the medical device according to claim 1; and
a package that packages the medical device, wherein
the package is subjected to sterilization treatment in a state where the medical device in which the inserter is disposed in advance between the expansion body and the handheld operation portion is packaged by inserting the shaft portion into the lumen of the inserter.

7. A method for using a medical device including:
an elongated shaft portion;
a handheld operation portion disposed at a proximal portion of the shaft portion;
an expansion body disposed at a distal portion of the shaft portion; and
an inserter including a lumen through which the shaft portion is inserted and which is capable of housing the expansion body, wherein
the inserter is disposed in advance between the expansion body and the handheld operation portion in a state where the shaft portion is inserted, and
the inserter is moved from a proximal side to a distal side to draw the expansion body into a distal opening of the inserter, and the expansion body is contracted and housed in the lumen of the inserter.

8. A method for manufacturing a medical device including:
an elongated shaft portion;
a handheld operation portion disposed at a proximal portion of the shaft portion;
an expansion body disposed at a distal portion of the shaft portion; and
an inserter including a lumen through which the shaft portion is inserted and which is capable of housing the expansion body, the method comprising:
inserting the shaft portion to which the expansion body is attached at the distal portion into the lumen of the inserter from a distal side of the inserter; and
attaching a shaft connecting portion for connecting the shaft portion to the handheld operation portion to the proximal portion of the shaft portion.

9. A method for manufacturing a medical device including:
an elongated shaft portion;
a handheld operation portion disposed at a proximal portion of the shaft portion;
an expansion body disposed at a distal portion of the shaft portion; and
an inserter including a lumen through which the shaft portion is inserted and which is capable of housing the expansion body, the method comprising:
inserting the shaft portion to which a shaft connecting portion for connecting the shaft portion to the handheld operation portion is attached at the proximal portion into the lumen of the inserter from a proximal side of the inserter; and
attaching the expansion body to the distal portion of the shaft portion.

10. A method for manufacturing a medical device including:
an elongated shaft portion;
a handheld operation portion disposed at a proximal portion of the shaft portion;
an expansion body disposed at a distal portion of the shaft portion; and
an inserter including a lumen through which the shaft portion is inserted and which is capable of housing the expansion body, the method comprising:
inserting the shaft portion into the lumen of the inserter; and
attaching a shaft connecting portion for connecting the shaft portion to the handheld operation portion to the proximal portion of the shaft portion inserted into the lumen of the inserter, and attaching the expansion body to the distal portion of the shaft portion inserted into the lumen of the inserter.
